# EUROPEAN PATENT SPECIFICATION

(11) **EP 2 624 711 B1**
(45) Date of publication and mention of the grant of the patent: **15.07.2015**
(21) Application number: 11807981.3
(22) Date of filing: 07.10.2011
(51) Int. Cl.: A23L 1/304, A23L 1/307, A61K 33/06

(54) **DIETARY FOOD PRODUCT WITH REDUCED FAT ABSORPTION**
DIÄTETISCHES LEBENSMITTELPRODUKT FÜR REDUZIERTE FETTRESORPTION
PRODUIT ALIMENTAIRE AVEC ABSORPTION DES GRAISSES RÉDUITE

(30) Priority: 08.10.2010 CA 2716718
(43) Date of publication of application: 14.08.2013
(73) Proprietor: Medicocensus GmbH, 30625 Hannover (DE)
(72) Inventor: ALBRECHT, Uwe, 31303 Burgdorf (DE)
(74) Representative: Thiel, Christian
(86) International application number: PCT/IB2011/002826
(87) International publication number: WO 2012/046142

(56) References cited:
- GB-A- 2 266 217
- US-A- 6 093 423
- US-A1- 2007 110 884
- US-B1- 6 803 064
- DATABASE FSTA [Online] INTERNATIONAL FOOD INFORMATION SERVICE (IFIS), FRANkFURT-MAIN, DE; 1999, MURATA T ET AL: "Inhibitory effect of calcium (derived from eggshell)-supplemented chocolate on absorption of fat in human males.", XP002669272, Database accession no. FS-1999-05-Ka0082 & MURATA T ET AL: "Inhibitory effect of calcium (derived from eggshell)-supplemented chocolate on absorption of fat in human male", JOURNAL OF JAPANEESE SOCIETY OF NUTRITION AND FOOD SCIENCE, vol. 51, no. 4, 1998, page 165, JOURNAL OF JAPANESE SOCIETY OF NUTRITION AND FOOD SCIENCE (NIPPON EIYO SHOKURYO GAKKAISHI) 1998 DEP. OF PAEDIATRICS, OSAKA MED. COLL., TAKATSUKI 569-8686, JAPAN
- DATABASE FSTA [Online] INTERNATIONAL FOOD INFORMATION SERVICE (IFIS), FRANkFURT-MAIN, DE; 2007, LORENZEN J K ET AL: "Effect of dairy calcium or supplementary calcium intake on postprandial fat metabolism, appetite, and subsequent energy intake.", XP002669273, Database accession no. FS-2007-09-Pa2283 & LORENZEN ET AL.: "Effect of dairy calcium or supplementary calcium intake on postprandial fat metabolism, appetite, and subsequent energy intake.", AMERICAN JOURNAL OF CLINICAL NUTRITION, vol. 85, no. 3, 2007, page 678, AMERICAN JOURNAL OF CLINICAL NUTRITION 2007 DEP. OF HUMAN NUTR., FAC. OF LIFE SCI., UNIV. OF COPENHAGEN, FREDERIKSBERG, DENMARK
- DATABASE BIOSIS [Online] BIOSCIENCES INFORMATION SERVICE, PHILADELPHIA, PA, US; 1994, KAMIWAKI TATUYA ET AL: "Absorption of fat of calcium-added chocolate", XP002669274, Database accession no. PREV199598076849 & KAMIWAKI TATUYA ET AL: "Absorption of fat of calcium-added chocolate", JOURNAL OF THE JAPANESE SOCIETY FOR FOOD SCIENCE AND TECHNOLOGY, vol. 41, no. 11, 1994, pages 763-767, ISSN: 0029-0394

## Description

### Field of the Invention

This invention relates to a method for reducing fat absorption by the human body when consuming a dietary commercial food product or meal according to the invention.

People who wish to reduce their body weight usually go on a low calorie diet, i.e., they reduce their calorific intake. Since fats or lipids are a highly concentrated source of energy and, therefore include significant calories, most diets aim to reduce the amount of fat consumed. However, low fat diets are often uninteresting and unappetizing and quite often a dieter will relapse from his diet and consume a high fat snack or meal.

### State of the Art

Attempts have been made to produce dietetic products with reduced fat absorption in the body with some producing a positive effect on blood cholesterol.

From GB 2 266 217 A a low calorie chocolate is known having a cholesterol reducing effect. The chocolate contains a calcium and/or magnesium salt and 2-unsaturated-1,3-disaturated triglyceride in a molar ratio of from 0.5:1 to 2:1. Calcium salts include calcium carbonates, calcium gluconic acids, calcium phosphoric acids, calcium milk serum, egg shell meal, bone meal and fish meal. Magnesium salts include magnesium carbonate. The salts are believed to associate with the fatty acids.

US 6 093 423 A discloses the use of a chemical agent, especially calcium sulphate, as a medicament. The patent provides a substance which may be taken after the consumption of a fatty snack or meal which will reduce the amount of fat absorbed from the fatty snack or meal and thus may provide relief to people on low fat diets when they have a temporary relapse. The chemical agent (calcium sulphate) binds to and insolubilises the products of fat digestion. This occurs in the environment of the small intestine so that the fat reaches the rectum and is excreted accordingly, thus preventing its absorption.

Dietary triacylglycerol is the major source of dietary fat for Western men. The digestion of triacylglycerol occurs mainly in the small intestine into which flow both bile and the secretions of the pancreas. Pancreatic lipase breaks down triacylglycerol into monoacylglycerol and fatty acids which, together with the bile salts, form soluble mi-celles diffuse across the "unstirred water layer" of the small intestine and come into contact with the microvillus membrane of the absorptive cells. Here the micelles dissociate to produce locally high concentrations of monoacylglycerol and fatty acids which are absorbed while the bile salts remain in the lumen to be absorbed in the lower regions of the intestine.

The chemical agent proposed by US 6,093,423 is supposed to act by dissociating and/or preventing micellar formation by displacing sodium or potassium ions from the micellar complexes. All this takes place in the small intestine or the gut. Since it was believed to be necessary that the chemical agent comes into contact with the free fatty acids produced during digestion of the fatty meal in the intestine, it was deemed to be essential to administer the medicament with the chemical agent immediately after or within two hours after consumption of the said fatty meal or snack.

It was found, however, that the action of the chemical agent in the body is not well characterized and that several side effects may occur. To achieve the desired effect it is necessary to take relatively high doses of the medicament. Artificial steatorrhoea may cause bacterial overgrowth and the problems associated therewith.

T. Murata et al., "Inhibitory effect of calcium (derived from eggshell) - supplemented chocolate on absorption of fat in human males", data base FS-1999-05-Ka 0082, J.K. Lorenzen et al., "Effect of dairy calcium or supplementary calcium intake on postprandial fat metabolism, appetite, and subsequent energy intake", data base FS-2007-09-Pa2283 and Tatuya Kamiwaki et al., "Absorption of fat of calcium-added chocolate", data base excession

PREV199598076849 desclares the use of calcium carbonate in order to inhibit the adsorption of fatty acids.

US 6,803,464 D1 and US 2007/110884 A1 disclose food compositions comprising calcium sulfate in an amount above 1/100 of the weight of the products.

None of the above cited documents discloses the use of calcium sulfate in a food for reducing fat absorption in the human body.

### Summary of the Invention

It is an object of the invention to provide means or a method to reduce the calorific intake and the absorption of fat from fatty foods.

These objects are achieved with the method of claim 1 and the use of claim 5, which comprise the use of calcium sulphate in one of its cristallization forms (including CaSO₄ • nH₂O) mixed with a commercial food product or meal when a portion of such a food or meal is consumed.

Certain exemplary embodiments may provide a commercial food product or meal comprising a dose of calcium sulphate wherein the minimum content of calcium sulphate is 1/100 of the total net weight of the food product or meal.

Yet another exemplary embodiment provides the use of a portion of food comprising a dose of calcium sulphate to reduce fat absorption by the human body, wherein the portion of food is consumed by the human body.

### Detailed Description

According to one exemplary embodiment there is provided a method for reducing fat absorption by the human body comprising the step of consuming a portion of food, i.e., a meal or a snack, preferably a dietary meal, wherein the portion of food comprises a dose of calcium sulphate.

In a preferred embodiment the ingredient calcium sulphate is intimately mixed with the food and spread all over its volume. In another embodiment the calcium sulphate is mixed with a fatty component of a multi-component food product. After that the fatty component is processed as usual within the multi-component food product.

The portion of food with the calcium sulphate distributed in it may be packaged.

Preferably the dose of calcium sulphate is at least equal by weight to half up to three quarters of the amount of a fat contained in the portion of food.

It is also preferred that the dose of calcium sulphate is at least equal to 1/100 part of the total net weight of the portion of food.

In a preferred embodiment the food is a multi-component food product, especially a convenience food product or a dietetic meal.

It is preferred that the food product to be consumed is a multi-component food product containing at least one fatty component, but the fat content should be reduced in the food or not too high as such.

Preferably the food product to be consumed according to the method of this invention is selected from the group consisting of food containing any meat, processed meat or dairy product, including casseroles, pizzas, pasta, cheese pastry, cheese, sausage, soups, especially tinned or in the form of a frozen food.

According to another aspect of the present invention there is provided a commercial food product or meal comprising a dose of calcium sulphate wherein the minimum content of calcium sulphate is 1/100 part of the total net weight of the food product or meal.

Preferably the dose of calcium sulphate in the commercial food product is at least equal by weight of from half up to three quarters of the amount of a fat contained in the portion of food.

The food of the commercial food product or meal is preferably a multi-component food product, especially a convenience food product or a dietetic meal. The fat content should be reduced in the food or be not too high as such. Preferably there is at least one fatty component. The calcium sulphate should be spread throughout the food product or meal or is premixed with the fatty component in the meal.

The commercial food product or meal is preferably selected from the group consisting of food containing any meat, processed meat or dairy product, including casseroles, pizzas, pasta, cheese pastry, cheese, sausage, soups, especially tinned or in the form of a frozen food. Preferably it is all natural food with a natural fat content. A reduced fat content in the meal is desirable to keep the content of calcium sulphate low.

The dairy products within the food or meal may be any dairy products except milk powder which should be excluded. The dairy products can for instance be selected from the group consisting of hard cheese, butter cheese, cotton cheese, curd cheese, sour milk and other processed milk.

All eatable fats can be present, such as butter, vegetable oil, margarine, lard, tallow, hard vegetable fat and so on.

Calcium sulphate is suited since it is eatable, non-toxic, of neutral taste, not affecting the mouth feel of the food or meal too much and poorly soluble in the aqueous medium of the meal or food.

It is important that the salt ingredient calcium sulphate gets into close contact with all of the fat in the meal, so it can act on all of the fat when digestion starts without having too much impact on the intestine itself and its flora where no fat is present.

It is supposed that according to certain embodiment calcium sulphate acts in the upper part of the digestive system and bind to the fats in the chymus. This is partly an adsorption or loose association of fats on the insoluble solid salts. Ion exchange starts with digestion and produces insoluble fat complexes with Ca-Ions. The intake of calories is thus restricted. The absorption of fats is depressed. Liver and gall are given more rest.

The commercial food product or meal can be contained in any container.

## Claims

1. A method for reducing fat absorption by the human body comprising the step of consuming a portion of food, wherein the portion of food comprises a dose of calcium sulphate, wherein the calcium sulphate is intimately mixed with the food and spread all over its volume.

2. The method according to claim 1 wherein the dose of calcium sulphate is at least equal by weight of from half to three quarters of the amount of a fat contained in the portion of food.

3. The method according to claim 1 or 2 wherein the dose of calcium sulphate is at least equal to 1/100 of the total weight of the portion of food.

4. The method of any one of claims 1 to 3 wherein the food is a multi-component food product, especially a convenience food product or a dietetic meal.

5. Use of a dose of calcium sulphate within a portion of food to reduce fat absorption by the human body, wherein the calcium sulphate is intimately mixed with the food and spread all over its volume.

6. Use according to claim 5 wherein the dose of calcium sulphate is at least equal by weight of from half to three quarters of the amount of a fat contained in the portion of food.

7. Use according to claim 5 or 6 wherein the dose of calcium sulphate is at least equal to 1/100 of the total weight of the portion of food.

8. Use according to any one of claims 6 to 7 wherein the portion of food is a commercial food product.

9. Use according to any of the claims 5 to 8 wherein the commercial food product is a multi-component food product, preferably a convenience food product or a dietetic meal.

## Patentansprüche

1. Verfahren zur Reduzierung der Fettaufnahme durch den menschlichen Körper, welches dem Schritt der Aufnahme einer Nahrungsmittelportion einschließt, wobei die Nahrungsmittelportion eine Dosis Kalziumsulfat enthält, in der das Kalziumsulfat innig mit dem Nahrungsmittel vermischt ist und über dessen Gesamtvolumen verteilt ist.

2. Verfahren nach Anspruch 1, worin die Kalziumsulfatdosis gewichtsmäßig zumindest gleich der Hälfte bis drei Viertel eines in der Nahrungsmittelportion enthaltenen Fetts ist.

3. Verfahren nach Anspruch 1 oder 2, worin die Kalziumsulfatdosis wenigstens 1/100 des Gesamtgewichts der Nahrungsmittelportion ausmacht.

4. Verfahren nach einem der Ansprüche 1 bis 3, worin das Nahrungsmittel ein vielkomponentiges Nahrungsmittelprodukt, insbesondere ein Convenience-Nahrungsmittelprodukt oder ein diätetisches Mahl ist.

5. Verwendung einer Kalziumsulfatdosis innerhalb einer Nahrungsmittelportion zur Reduzierung der Fettabsorption durch den menschlichen Körper, worin das Kalziumsulfat innig mit dem Nahrungsmittel vermischt ist und über dessen Gesamtvolumen verteilt ist.

6. Verwendung nach Anspruch 5, worin die Kalziumsulfatdosis gewichtsmäßig wenigstens gleich der Hälfte bis drei Viertel der Menge eines in der Nahrungsmittelportion enthaltenden Fetts ist.

7. Verwendung nach Anspruch 5 oder 6, worin die Kalziumsulfatsdosis wenigstens 1/100 des Gesamtgewichts der Nahrungsmittelportion ausmacht.

8. Verwendung nach einem der Ansprüche 6 bis 7, worin die Nahrungsmittelportion ein kommerzielles Nahrungsmittelprodukt ist.

9. Verwendung nach einem der Ansprüche 5 bis 8, worin das kommerzielle Nahrungsmittelprodukt ein vielkomponentiges Nahrungsmittelprodukt, vorzugsweise ein Convenience-Nahrungsmittelprodukt oder ein diätetisches Mahl ist.

## Revendications

1. Procédé de réduction de l'absorption de graisses par le corps humain, comprenant l'étape de consommer une portion d'un aliment, la portion d'aliment comprenant une dose de sulfate de calcium, le sulfate de calcium étant intimement mélangé avec l'aliment et réparti sur son volume entier.

2. Procédé selon la revendication 1, **caractérisé en ce que** la dose de sulfate de calcium est au moins égale en poids à la moitié jusqu'à trois quarts du montant d'une graisse contenue dans la portion d'aliment.

3. Procédé selon la revendication 1 ou 2, **caractérisé en ce que** la dose de sulfate de calcium est au moins égale à un centième du poids total de la portion d'aliment.

4. Procédé selon l'une des revendications 1 à 3, **caractérisé en ce que** l'aliment est un produit alimentaire multi-composants, notamment un plat préparé ou un repas diététique.

5. Utilisation d'une dose de sulfate de calcium dans une portion d'un aliment pour réduire l'absorption de graisses par le corps humain, le sulfate de calcium étant intimement mélangé avec l'aliment et réparti sur son volume entier.

6. Utilisation selon la revendication 5, **caractérisée en ce que** la dose de sulfate de calcium est au moins égale en poids à la moitié jusqu'à trois quarts du montant d'une graisse contenue dans la portion d'aliment.

7. Utilisation selon la revendication 5 ou 6, **caractérisée en ce que** la dose de sulfate de calcium est au moins égale à un centième du poids total de la portion d'aliment.

8. Utilisation selon l'une quelconque des revendications 6 à 7, **caractérisé en ce que** la portion d'aliment est un produit alimentaire du commerce.

9. Utilisation selon l'une quelconque des revendications 5 à 8, **caractérisé en ce que** le produit alimentaire du commerce est un produit alimentaire multi-composants, de préférence un plat préparé ou un repas diététique.
